# EUROPÄISCHE PATENTANMELDUNG

(11) **EP 0 543 134 A1**
(43) Veröffentlichungstag der Anmeldung: **26.05.1993**
(21) Anmeldenummer: 92117278.9
(22) Anmeldetag: 09.10.1992
(51) Int. Cl.: A61L 2/20

(54) **Verfahren und Anlage zur Rückgewinnung eines Sterilisiergases**

(30) Priorität: 21.11.1991 DE 4138321
(71) Anmelder: HERCO KÜHLTECHNIK HERMANNS & CO. GmbH, D-46485 Wesel (DE); AIR PRODUCTS GMBH, D-45527 Hattingen (DE)
(72) Erfinder: Karthaus, Michael, Dipl.-Ing., W-4040 Neuss (DE); Hermanns, Klaus, Dr. rer. nat., W-4224 Hünxe 2 (DE); Hermanns, Peter, Dr., W-4230 Wesel (DE)
(74) Vertreter: Marx, Lothar, Dr.

(57) **Zusammenfassung**

Bei einem Verfahren und einer Vorrichtung zur Rückgewinnung eines Sterilisiergases, insbesondere von Ethylenoxid (ETO), werden zu sterilisierende Materialien in einer Sterilisierkammer (1) mit dem Sterilisiergas sterilisiert und ein Gasstrom aus der Sterilisierkammer (1) abgezogen. Zumindest ein Teil des in diesem Gasstrom enthaltenen Sterilisiergases wird verflüssigt und abgeschieden Der vom verflüssigten Sterilisiergas befreite Gasstrom wird in die Sterilisierkammer (1) zurückgeführt, während gleichzeitig zumindest ein Teil des abgeschiedenen Sterilisiergases unter Reduzierung der Konzentration des Sterilisiergases in der Sterilisierkammer (1) durch Zugabe von inertisierendem Gas ersetzt wird.

## Beschreibung

Die Erfindung betrifft ein Verfahren und eine Anlage zur Rückgewinnung eines Sterilisiergases, insbesondere von Ethylenoxid, bei dem zu sterilisierende Materialien in einer Sterilisierkammer mit dem Sterilisiergas sterilisiert werden und ein Gasstrom aus der Sterilisierkammer abgezogen und zumindest ein Teil des darin enthaltenen Sterilisiergases aus dem Gasstrom abgeschieden wird.

Als Sterilisiergase finden üblicherweise Alkylenoxide, insbesondere Ethylenoxid, die zumeist mit inertisierenden Gasen wie beispielsweise Freon (Dichlordifluormethan) oder Inertgasen, insbesondere Stickstoff, gemischt werden, Verwendung. Aus Kosten- und Umweltverträglichkeitsgründen werden die Sterilisiergasgemische, d.h. das eigentliche Sterilisiergas und die inertisierenden weiteren Bestandteile nach einem Sterilisiervorgang nicht einfach in die Umgebung abgelassen, sondern zurückgewonnen und wiederverwendet.

So gehen aus der US-PS 3 549 312 ein Verfahren sowie eine Anlage zur Rückgewinnung von Alkylenoxid und den beigemischten inerten Bestandteilen hervor, bei denen das aus einer Sterilisierkammer abgesaugte Gemisch von Alkylenoxid und den inerten Beimischungen einem Vorkühler und anschließend einem Absorber zugeführt werden. Das in dem Absorber von Feuchtigkeit befreite Gemisch gelangt dann in einen Kondensator zur Verflüssigung des Alkylenoxids und des beigemischten Freon R 12 und schließlich in ein Reservoir zur Zwischenspeicherung des flüssigen Gemisches. Aus dem unter Überdruck stehenden Reservoir kann dieses Gemisch schließlich wieder der Sterilisierkammer zugeführt werden. Nachteilig wirkt sich bei diesem Rückgewinnungsverfahren aus, daß die Sterilisierkammer nach einem Sterilisierzyklus ausgepumpt wird, um das Gas bzw. Gasgemisch dem angeschlossenen Rückgewinnungsteil der Anlage zuführen und im nächsten Sterilisierzyklus wiederverwenden zu können. Bei einem Rückgewinnungsverfahren nach der US-PS 3 549 312 wird beim Abpumpen des Sterilisiergases bzw. des Gasgemisches gleichzeitig und im gleichen Maße Umgebungsluft in die Sterilisierkammer eingelassen. Obwohl die eingelassene Luft und das abzupumpende Sterilisiergas bzw. das Gasgemisch große Dichteunterschiede aufweisen und daher auf natürliche Weise voneinander getrennt bleiben, kann nicht ausgeschlossen werden, daß insbesondere zum Ende des Abpumpvorgangs hin in steigendem Maße unerwünschterweise Umgebungsluft in die Rückgewinnungsanlage gelangt. Diese Gefahr ist gerade deswegen gegeben, weil die Sterilisierkammer bis auf geringe Sterilisiergasreste abgepumpt werden muß, um sie anschließend für den nächsten Sterilisierzyklus öffnen zu können.

Nach einem durch die EP-A 0 130 319 offenbarten Verfahren zur Reinigung von Ethylenoxid oder einem Gemisch aus Ethylenoxid und einem fluorierten Kohlenwasserstoff wird die während des Sterilisierens unter Überdruck stehende Sterilisierkammer nach jedem Sterilisierprozeß zuerst druckentlastet und danach bis auf einen Druck zwischen 10 und 100 mbar mit Hilfe einer ölfrei arbeitenden Vakuumpumpe evakuiert. Die abgelassenen und anschließend abgesaugten Gase werden gereinigt und zurückgewonnen, um für einen neuen Sterilisiervorgang zur Verfügung zu stehen. Durch diese Restevakuierung auf zumindest 100 mbar soll der Sterilisiergasverlust auf einem erträglichen Niveau gehalten werden. Bei einer nach diesem Verfahren arbeitenden Anlage sind innerhalb des Rückgewinnungsteils der Sterilisieranlage aufwendige Pump- und Kühlsysteme zum Evakuieren der Sterilisierkammer notwendig. Desweiteren ändert sich während der Rückgewinnung selbst ständig der Druck in den Komponenten und Leitungssystemen des Rückgewinnungsteils vom über dem Atmosphärendruck liegenden Ausgangsdruck in der Kammer bis auf den Druck von 10 bis 100 mbar.

Ein Verfahren und eine Anlage zur Rückgewinnung eines Sterilisiergases, gemäß dem das Sterilisiergas nach einem Sterilisierprozeß ebenfalls aus der Sterilisierkammer abgepumpt und in einem Rückgewinnungskreislauf zurückgewonnen wird, geht aus der nicht vorveröffentlichten deutschen Patentanmeldung Nr. 41 17 306.6 hervor.

Ein weiteres Verfahren und eine Einrichtung zur Rückgewinnungen mindestens einer Komponente eines Sterilisiergases geht aus der EP-A-0 326 985 hervor. Um das Gasgemisch aus der Sterilisierkammer abzulassen, wird im Falle eines nahe bei Atmosphärendruck liegenden Kammerdrucks vorgeschlagen, ein Gas, beispielsweise Stickstoff, bei einem höheren Druck in die Sterilisierkammer zu drücken und das Sterilisiergas dadurch auszutreiben. Dabei wird der Kammerdruck zumindest zeitweise oberhalb des Atmosphärendrucks liegen, wodurch die Gefahr von Leckverlusten, d.h. Austritt von toxischem Sterilisiergas in die Umgebung, erhöht wird. Als weiterer Nachteil ist der hohe Stickstoffverbrauch beim Austreiben des Sterilisiergases anzusehen.

Desweiteren ist es aus der EP-A-0 417 592 bekannt, bei der Rückgewinnung von Lösungsmitteln, die in kontinuierlich ablaufenden Fertigungsprozessen, beispielsweise beim Trocknen beschichteter Video- oder Audiobänder, anfallen, den Trockner mit einem inerten Gas zu befüllen und die bei der Trocknung entstehenden Lösungsmitteldämpfe zusammen mit diesem inerten Gas in einem Lösungsmittelrückgewinnungskreislauf zurückzugewinnen und das von Lösungsmitteldämpfen befreite Gas wieder in den Trockner zurückzuführen. Bei einer Abfahreinrichtung für solch eine Lösungsmittelrückgewinnungsanlage, wie sie aus der nicht vorveröffentlichten deutschen Patentanmeldung P 40 40 389 hervorgeht, kann dem Trockner nicht nur über den Lösungsmittelkreislauf, sondern über eine zusätzliche Ab- und eine zusätzliche Zuleitung Gemisch entzogen und gleichzeitig reines Inertgas zugeführt werden. Damit wird das Ziel verfolgt, im Falle von Störungen im Trockner dessen Gasatmosphäre innerhalb kürzester Zeit wieder auf Normalwerte bringen oder noch innerhalb der zulässigen Werte halten zu können.

Die vorliegende Erfindung hat es sich zur Aufgabe gemacht, die bei den aus dem Stand der Technik bekannten Verfahren und Anlagen zur Rückgewinnungen eines Sterilisiergases auftretenden Nachteile zu vermeiden. Dabei soll insbesondere auf aufwendige Evakuierungssysteme innerhalb der Rückgewinnungsanlage verzichtet, die Betriebssicherheit beim Entfernen des Sterilisiergases aus der Sterilisierkammer und bei dessen Rückgewinnung erhöht und die für die Sterilisiergasrückgewinnung notwendige Inertgasmenge reduziert werden.

Diese Aufgabe wird durch den Gegenstand des Anspruchs 1 gelöst.

Weitere vorteilhafte und zweckmäßige, nicht glatt selbstverständliche Ausgestaltungen der Erfindung werden durch die Unteransprüche offenbart.

Bei einem Verfahren und einer Anlage zur Rückgewinnung eines Sterilisiergases, insbesondere von Ethylenoxid (ETO), werden zu sterilisierende Materialien in einer Sterilisierkammer mit dem Sterilisiergas sterilisiert und ein Gasstrom aus der Sterilisierkammer abgezogen. Zumindest ein Teil des in diesem Gasstrom enthaltenen Sterilisiergases wird abgeschieden. Erfindungsgemäß wird der vom abgeschiedenen Sterilisiergas befreite Gasstrom in die Sterilisierkammer zurückgeführt, während gleichzeitig zumindest ein Teil des abgeschiedenen Sterilisiergases unter Reduzierung der Konzentration des Sterilisiergases in der Sterilisierkammer durch Zugabe von inertisierendem Gas ersetzt wird.

Indem gleichzeitig auf der einen Seite Sterilisiergas, d.h. reines Sterilisiergas oder ein das Sterilisiergas enthaltendes Gasgemisch, aus der Sterilisierkammer abgezogen und auf der anderen Seite der vom abgeschiedenen Sterilisiergas befreite Gasstrom in die Sterilisierkammer zurückgeführt wird, läßt sich eine kontinuierliche Sterilisiergasrückgewinnung mit kontinuierlich sinkenden Mengen an abgeschiedenem Sterilisiergas sowie kontinuierlicher Neutralisierung der Sterilisierkammer erzielen. Damit der Druck in der Sterilisierkammer und ganz besonders auch im Rückgewinnungskreislauf nicht oder zumindest nicht zu stark sinkt, wird zumindest ein Teil des abgeschiedenen Sterilisiergases durch Zugabe von inertisierendem Gas ersetzt. Durch die Zugabe solch eines Gases, das bevorzugterweise ein Inertgas und besonders bevorzugt Stickstoff ist, wird darüberhinaus die Sterilisierkammer vorteilhaft gespült. Dabei wird die Rückgewinnungsanlage durch die dosierte Zugabe des inertisierenden Gases, wobei in diesem Zusammenhang stellvertretend nur noch von Stickstoff die Rede sein soll, nicht durch übergroße Stickstoffmengen unnötig belastet. Dies ist jedoch der Fall, wenn Stickstoff zum Austreiben des Sterilisiergases einfach in die Kammer gedrückt wird. Gegenüber solch einem Verfahren wird Stickstoff eingespart. Auf aufwendige Evakuierungssysteme im Rückgewinnungskreislauf kann verzichtet werden.

Gemäß einer bevorzugten Ausführungsform der Erfindung erfolgt die Dosierung des zugegebenen Stickstoffs in Abhängigkeit vom Druck in der Sterilisierkammer. Das Sterilisiergas in der Sterilisierkammer wird besonders bevorzugt kontinuierlich, d.h. bei sinkenden Zugaberaten des Stickstoffs ausgetauscht, bis die Konzentration des Sterilisiergases in der Kammer unterhalb eines vorbestimmten Wertes gesunken ist Zweckmäßigerweise liegt dieser Wert bei oder unterhalb des Wertes, der einer ungefährlichen Restgaskonzentration entspricht, so daß die Sterilisierkammer zum Beladen mit neuen Materialien für den nächsten Sterilisierzyklus geöffnet werden kann.

Um den Rückgewinnungskreislauf für das Sterilisiergas besonders effektiv betreiben zu können, aber auch um ein Höchstmaß an Sicherheit mit Blick auf nie ganz auszuschließende Leckagen zu erreichen, läuft das erfindungsgemäße Verfahren bei in etwa konstantem Kammerdruck ab. Es wird also immer in etwa so viel zusätzlicher Stickstoff zur Sterilisierkammer geführt, wie zur Kompensation des abgeschiedenen Sterilisiergases im Sinne der Aufrechterhaltung des Drucks in der Kammer notwendig ist. Das abgeschiedene Sterilisiergas wird kontinuierlich, sozusagen auf iterativem Wege, durch Stickstoff ersetzt. Ganz besonders bevorzugt liegt dieser aufrechtzuerhaltende Kammerdruck in der Nähe des Umgebungsdrucks der Sterilisierkammer bzw. der Rückgewinnungsanlage, der im allgemeinen dem Atmosphärendruck entspricht. Damit wird die Gefahr eines Sterilisiergasaustritts aus der Anlage und eines Sauerstoffeintritts in die Anlage in Folge von Leckagen, z.B. Rissen, weitgehend ausgeschaltet.

Der Rückgewinnungskreislauf für das Sterilisiergas umfaßt einen Trocknungsteil und einen Tieftemperaturteil. Im Trocknungsteil wird der aus der Sterilisierkammer abgezogene Gasstrom von Feuchtigkeit befreit und im Tieftemperaturteil wird ein Teil des im getrockneten Gasstrom enthaltenden Sterilisiergases verflüssigt und abgeschieden. Zwischen dem Trocknungsteil und dem Tieftemperaturteil des Rückgewinnungskreislaufs ist gemäß einer bevorzugten Ausführungsform der Erfindung ein Wärmetauscher angeordnet, der vorteilhaft dazu dient, einen Wärmeaustausch zwischen dem Gasstrom, der sich infolge der Adsorption von Feuchtigkeit in einem Adsorber des Trocknungsteils erwärmt hat, und dem vom abgeschiedenen Sterilisiergas befreiten Gasstrom, der in die Sterilisierkammer zurückgeführt wird, Zu bewirken.

Durch die erfindungsgemäße Anordnung eines solchen Rekuperators wird zum einen die erforderliche Kälteleistung des Tieftemperaturteils zur Verflüssigung von Sterilisiergas verringert und zum anderen das in die Sterilisierkammer zurückzuführende Restgas vorerwärmt.

Für die Rückführung des Restgases in die Sterilisierkammer genügt, wegen der bei dem erfindungsgemäßen Verfahren geringen Druckunterschiede, ein Ventilator zur Aufrechterhaltung des Rückgewinnungskreislaufs.

Die Zugabe des das abgeschiedene Sterilisiergas ersetzenden Stickstoffs erfolgt gemäß der Erfindung über ein steuerbares Dosiermittel, insbesondere ein steuerbares Dosierventil. Gesteuert wird dieses Dosiermittel durch einen Regler in Abhängigkeit von einem oder mehreren in der Sterilisierkammer angeordneten Druckfühlern. Dazu ist bevorzugterweise ein Proportional-Integral(PI)-Regler vorgesehen, so daß die Steuerung des Dosiermittels bereits mit einem gewissen Vorhalt auf sich ändernde Druckverhältnisse innerhalb der Sterilisierkammer erfolgt.

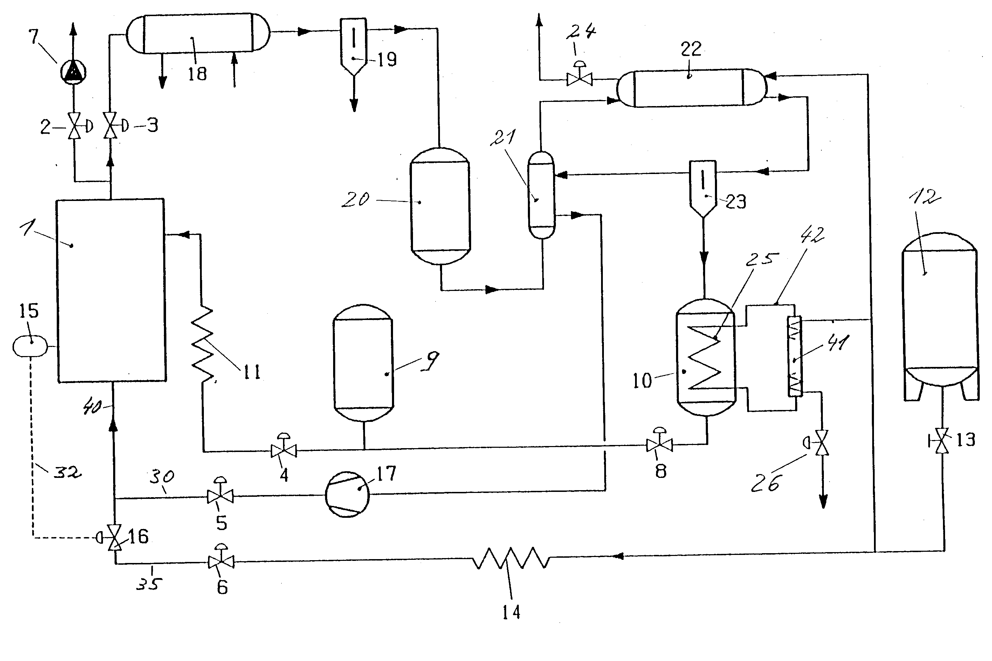

Eine besonders zweckmäßige Ausführungsform der Erfindung wird im folgenden anhand der Figur beschrieben. Dabei werden weitere Merkmale und Vorteile der Erfindung offenbart.

Die Figur zeigt den schematischen Aufbau einer Anlage zur Rückgewinnung eines Sterilisiergases. Nach dem Beladen mit zu sterilisierenden Materialien wird eine Sterilisierkammer 1 zunächst bei geöffnetem Ventil 2 und geschlossenen Ventilen 3, 4, 5 und 6 durch eine Vakuumpumpe 7 evakuiert. Anschließend wird die Sterilisierkammer 1 nach Schließen des Ventils 2 mit einem Gemisch aus Ethylenoxid (ETO) und Stickstoff in der Zusammensetzung von etwa 40 % ETO und 60 % Stickstoff geladen.

Es sei vermerkt, daß zur Sterilisierung auch reines ETO oder eine Mischung aus ETO und Stickstoff, insbesondere mit einem ETO-Anteil von 10 bis 60 % und einem entsprechenden Stickstoff-Anteil von 40 bis 90%, verwendet werden kann. Grundsätzlich sind zur Inertisierung des ETO's auch andere Gase geeignet. Stickstoff nimmt dabei jedoch aufgrund seiner leichten Handhabbarkeit und Wirtschaftlichkeit sowie seiner Umweltverträglichkeit eine herausragende Stellung ein.

Das Auffüllen der Sterilisierkammer 1 mit Sterilisiergas wird durch öffnen der Ventile 4 und gegebenenfalls 8 sowie anschließendes öffnen der Ventile 6 und 13 eingeleitet. Dadurch gelangt ETO aus einem unter Eigendruck stehenden ETO-Tank 9 bzw. aus einem ebenfalls unter Eigendruck stehenden ETO-Puffer 10 über einen ETO-Verdampfer 11 in die Sterilisierkammer 1, während Stickstoff aus einem Tank 12 für Flüssigstickstoff über einen Stickstoffverdampfer 14, Zuführungen 35 und 40 sowie ein steuerbares Ventil 16 zur Sterilisierkammer 1 geführt wird. Nach Füllung der Sterilisierkammer 1 mit dem ETO oder dem ETO/Stickstoff-Gemisch soll der Innendruck der Sterilisierkammer 1 etwa dem Umgebungsdruck entsprechen. Jetzt wird das Ventil 4 geschlossen. Das Ventil 16 wird durch einen Regler 15 in Abhängigkeit vom Druck in der Sterilisierkammer 1 über eine Steuerleitung 32 so gesteuert, daß in der Sterilisierkammer 1 stets annähernd Umgebungsdruck herrscht.

Nach ausreichender Einwirkzeit des ETO auf die zu sterilisierenden Materialien ist der Sterilisiervorgang beendet.

Zur Rückgewinnung des ETO werden jetzt die Ventile 3 und 5 geöffnet und ein Ventilator 17 in Betrieb genommen. Dadurch entsteht ein geschlossener Kreislauf für die Rückgewinnung des Sterilisiergases aus der Sterilisierkammer 1. Ein aus der Sterilisierkammer 1 abgezogener, das Sterilisiergas enthaltender Gasstrom wird dabei nacheinander über die folgenden Komponenten geführt: Vorkühler 18 - Wasserabscheider 19 - Trockner 20 - Rekuperator 21 - Tiefkühlkondensator 22 - ETO-Abscheider 23 - Rekuperator 21 - Ventilator 17 und Zuführungen 30, 40 zurück in die Sterilisierkammer 1. Der in diesem Kreislauf geführte Gasstrom hat keinerlei Verbindung zur Umgebung. Der Gasstrom aus der Sterilisierkammer 1 wird im Vorkühler 18 auf wenig mehr als 0°C, insbesondere auf ca. 4° bis 10°C, gekühlt. Der dabei kondensierte überwiegende Teil des im Gasstrom enthaltenen Wasserdampfes wird im nachfolgenden Wasserabscheider 19 abgeschieden. Der Gasstrom wird nun zu einem Trockner 20, der insbesondere als Adsorber, d.h. als Molekularsieb 20, ausgebildet ist, geführt. Aus dem bereits vorgetrockneten Gasstrom wird im Adsorber 20 nahezu die gesamte Restfeuchtigkeit entzogen. Der Taupunkt des den Adsorber 20 verlassenden Gasstroms liegt im Bereich zwischen -80°C und -100°C. Im Kreislauf wird ständig ETO im Tiefkühlkondensator 22 verflüssigt, die flüssige Phase in einem ETO-Abscheider 23 durch Abscheidung abgetrennt und in einem ETO-Puffer 10 in flüssiger Form gespeichert. Das durch die Abscheidung entfernte ETO wird über das vom Regler 15 gesteuerte Ventil 16 durch Stickstoff, der aus dem Flüssigstickstofftank 12 stammt, ersetzt, so daß der Druck in der Sterilisierkammer 1 und somit im gesamten Rückgewinnungskreislauf konstant gehalten wird. Dadurch können zumindest verfahrensbedingte Druckschwankungen im Kreislauf vermieden werden. Um auf sich verändernde Drücke in der Sterilisierkammer mit einem gewissen Vorhalt reagieren zu können, ist der Regler 15 als Proportional-Integral (PI)-Regler ausgebildet.

Während des Betriebs des Ventilators 17 sinkt der ETO-Anteil im Kreislauf bei gleichzeitigem Anstieg des Stickstoffanteils ständig weiter ab. Wenn der ETO-Anteil in der Sterilisierkammer 1 soweit gesunken ist, daß die behördlich zulässigen Grenzen unterschritten sind, werden die Ventile 3, 5 und 6 geschlossen und der Ventilator 17 abgeschaltet. Damit ist die Rückgewinnung beendet, und die Sterilisierkammer 1 kann geöffnet werden.

Neben dem der Sterilisierkammer 1 zugeführten Stickstoff aus dem Stickstofftank 12 wird aus dem gleichen Tank 12 Flüssigstickstoff zur Kühlung des Tiefkühlkondensators 22 abgezogen. Über ein stromaufwärts des Tiefkühlkondensators 22 angeordnetes Ventil 24 kann dessen Kühlleistung reguliert werden.

Ein wesentlicher Teil des aus dem Stickstofftank 12 stammenden Flüssigstickstoffs wird in einem Wärmetauscher 41 zur Kühlung eines Zwischenkälteträgerkreislaufs 42 mit z.B. R 22 auf z.B. ca. -85 bis -90°C benutzt, der seinerseits das im EO-Puffer gespeicherte ETO durch einen Wärmetauscher 25 auf z.B. ca. -80°C hält. Die Menge des hierfür erforderlichen Flüssigstickstoffs kann wiederum durch ein Ventil 26 reguliert werden.

Zwischen dem aus dem Vorkühler 18, dem Wasserabscheider 19 und dem Adsorber 20 gebildeten Trocknungsteil des Rückgewinnungskreislaufs und dem Tieftemperaturteil, der im wesentlichen den Tiefkühlkondensator 22 und den ETO-Abscheider 23 umfaßt, ist der Rekuperator 21 angeordnet. Hierdurch werden der noch warme, d.h. im als Molekularsieb ausgebildeten Adsorber 20 wieder erwärmte Gasstrom und der im Tiefkühlkondensator 22 abgekühlte und im ETO-Abscheider 23 vom abgeschiedenen ETO befreite Gasstrom im gegenseitigen Wärmeaustausch vorgekühlt bzw. vorerwärmt. Diese Art des Wärmerecyclings kommt der Energiebilanz der Rückgewinnung zugute, da hierdurch die zur Kühlung des Tiefkühlkondensators 22 benötigte Stickstoffmenge gering gehalten werden kann.

## Patentansprüche

1. Verfahren zur Rückgewinnung eines Sterilisiergases, insbesondere von Ethylenoxid (ETO), bei dem
a) zu sterilisierende Materialien in einer Sterilisierkammer mit dem Sterilisiergas sterilisiert werden, und
b) ein Gasstrom aus der Sterilisierkammer abgezogen und zumindest von einem Teil seines Sterilisiergases durch verflüssigung desselben und Abscheidung des verflüssigten Sterilisiergases in einem Abscheider (23) befreit wird,
**dadurch gekennzeichnet,** daß
c) der von dem verflüssigten Sterilisiergas befreite Gasstrom in die Sterilisierkammer (1) zurückgeführt wird; und
d) gleichzeitig zumindest ein Teil des verflüssigten Sterilisiergases unter Reduzierung der Konzentration des Sterilisiergases in der Sterilisierkammer (1) durch Zugabe von inertisierendem Gas ersetzt wird.

2. Verfahren nach Anspruch 1, dadurch gekennzeichnet, daß die Zugabe des inertisierenden Gases unter Aufrechterhaltung eines in etwa konstanten Drucks in der Sterilisierkammer (1) dosiert wird.

3. Verfahren nach einem der Ansprüche 1 und 2, dadurch gekennzeichnet, daß der Austausch des Sterilisiergases in der Sterilisierkammer (1) kontinuierlich bei sinkenden Zugaberaten des inertisierenden Gases erfolgt, bis die Konzentration des Sterilisiergases in der Sterilisierkammer (1) unterhalb eines vorbestimmten Wertes gesunken ist.

4. Verfahren nach einem der Ansprüche 1 bis 3, dadurch gekennzeichnet, daß der aus der Sterilisierkammer (1) abgezogene Gasstrom in einem Vorkühler (8) zur Kondensation eines großen Teils des mitgeführten Wasserdampfes gekühlt und durch einen Wasserabscheider (19) geleitet wird.

5. Verfahren nach Anspruch 4, dadurch gekennzeichnet, daß der in dem wasserabscheider (19) von einem großen Teil des mitgeführten Wasserdampfes befreite Gasstrom in einem Adsorber (20), der insbesondere ein Molekularsieb aufweist, von Restfeuchtigkeit befreit wird.

6. Verfahren nach einem der Ansprüche 1 bis 5, dadurch gekennzeichnet, daß der vom abgeschiedenen Sterilisiergas befreite Gasstrom vor der Rückführung in die Sterilisierkammer (1) durch Wärmeaustausch mit dem Gasstrom aus dem Adsorber (20) erwärmt wird.

7. Verfahren nach einem der Ansprüche 1 bis 6, dadurch gekennzeichnet, daß der vom abgeschiedenen Teil des Sterilisiergases befreite Gasstrom vor der Rückführung zur Sterilisierkammer (1) mit dem als Zugabe dienenden inertisierenden Gas zusammengeführt wird.

8. Verfahren nach einem der Ansprüche 1 bis 7, dadurch gekennzeichnet, daß als Sterilisiergas bzw. Sterilisiergasgemisch reines ETO oder ein Gemisch aus ETO und einem Inertgas, insbesondere Stickstoff, mit einem Anteil von 20 bis 50% ETO und entsprechend 50 bis 80 % Inertgas, insbesondere von etwa 40 % ETO und etwa 60 % Inertgas, verwendet wird.

9. Verfahren nach einem der Ansprüche 1 bis 8, dadurch gekennzeichnet, daß der Druck in der Sterilisierkammer in etwa dem Umgebungsdruck, insbesondere dem atmosphärischen Druck, entspricht.

10. Vorrichtung zur Rückgewinnung eines Sterilisiergases, insbesondere von Ethylenoxid (ETO),
a) mit einer Sterilisierkammer (1) zur Aufnahme der zu sterilisierenden Materialien,
b) mit einem Tieftemperaturkondensator (22) zur zumindest teilweisen Verflüssigung des in einem aus der Sterilisierkammer (1) abgezogenen Gasstrom enthaltenden Sterilisiergases, und
c) mit einem Abscheider (23) zum Abscheiden des verflüssigten Sterilisiergases,
**gekennzeichnet durch**
d) eine Rückführung (30) für den vom abgeschiedenen Sterilisiergas befreiten Gasstrom zur Sterilisierkammer (1);
e) eine zusätzliche Zuführung (35) für ein das Sterilisiergas inertisierendes Gas zur Sterilisierkammer (1); und
f) ein in der zusätzlichen Zuführung (35) angeordnetes, in Abhängigkeit von der abgeschiedenen Sterilisiergasmenge gesteuertes Dosiermittel (16) zum Dosieren des inertisierenden Gases.

11. Vorrichtung nach Anspruch 10, dadurch gekennzeichnet, daß zur Steuerung des als Dosierventil (16) ausgebildeten Dosiermittels ein Regler (15), insbesondere ein Proportional-Integral (PI)-Regler, vorgesehen ist, der das Dosierventil (16) in Abhängigkeit von dem in der Sterilisierkammer (1) herrschenden Druck steuert.

12. Vorrichtung nach Anspruch 10 oder 11, dadurch gekennzeichnet, daß zur Förderung des vom abgeschiedenen Sterilisiergas befreiten Gasstroms ein Ventiiator (17) in der Rückführung (30) zur Sterilisierkammer (1) vorgesehen ist.

13. Vorrichtung nach einem der Ansprüche 10 bis 12, dadurch gekennzeichnet, daß stromaufwärts von dem Tieftemperaturkondensator (22) ein Rekuperator (21) für den Wärmeaustausch zwischen dem Gasstrom zum Tieftemperaturkondensator (22) und dem Gasstrom vom Abscheider (23) angeordnet ist.

14. Vorrichtung nach einem der Ansprüche 10 bis 13, dadurch gekennzeichnet, daß stromaufwärts von dem Tieftemperaturkondensator (22) ein Adsorber (20), insbesondere ein Molekularsieb (20), durch den der Gasstrom aus der Sterilisierkammer (1) von Restfeuchtigkeit befreit wird, angeordnet ist.

15. Vorrichtung nach Anspruch 14, dadurch gekennzeichnet, daß der Rekuperator (21) in Strömungsrichtung zwischen dem Adsorber (20) und dem Tieftemperaturkondensator (22) einerseits und dem Abscheider (23) und der Sterilisierkammer (1) andererseits angeordnet ist.
